# EUROPÄISCHE PATENTSCHRIFT

(11) **EP 0 794 169 B1**
(45) Veröffentlichungstag und Bekanntmachung des Hinweises auf die Patenterteilung: **29.12.1999**
(21) Anmeldenummer: 97102368.4
(22) Anmeldetag: 14.02.1997
(51) Int. Cl.: C07C 69/68, C07C 67/62

(54) **Stabilisierter Milchsäurementhylester**
Stabilized menthyl lactate
Lactate de menthyle stabilisé

(30) Priorität: 27.02.1996 DE 19607278
(43) Veröffentlichungstag der Anmeldung: 10.09.1997
(73) Patentinhaber: HAARMANN & REIMER GMBH, D-37601 Holzminden (DE)
(72) Erfinder: Kuhn, Walter, Dr., 37603 Holzminden (DE); Körber, Alfred, Dr., 37603 Holzminden (DE); Langner, Roland, 37639 Bevern (DE); Hagena, Detlef, Dr., 37671 Stahle (DE)
(74) Vertreter: Petrovicki, Wolfgang, Dr.

(56) Entgegenhaltungen:
- Keine einschlägigen Dokumente gefunden

## Beschreibung

Milchsäurementhylester (nachfolgend: MME) 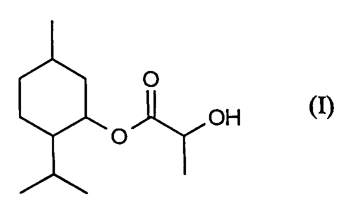 ist eine Verbindung mit physiologischer Kühlwirkung auf Haut- und Schleimhäute des Körpers, die in Konsumprodukten eingesetzt werden kann, bei denen eine lang anhaltende physiologische Kühlwirkung erwünscht wird: z.B. in Genußmitteln wie Kaugummi, Kautabak, Zigaretten, Eis, Konfekt, Getränken sowie in pharmazeutischen Präparaten, Körperpflegemitteln oder kosmetischen Präparat wie Zahnreinigungsmitteln, Mundwässern, Parfüms, Puder, Lotionen, Salben, Ölen, Cremes, Rasierwässern, Shampoos.

MME wird üblicherweise durch säurekatalysierte Veresterung handelsüblicher L-Milchsäure mit L-Menthol hergestellt.

Geeignete Veresterungskatalysatoren sind beispielsweise in Methoden der Organischen Chemie (Houben-Weyl), Bd. VIII, S. 517, Georg Thieme Verlag, Stuttgart 1952, beschrieben. Bevorzugte Katalysatoren umfassen anorganische Säuren wie Schwefelsäure, Phosphorsäure, saure Erden, organische Säuren wie Methansulfonsäure und p-Toluolsulfonsäure sowie saure Ionenaustauscher. Die empfohlenen Mengen betragen 0,0001-0,1 Mol, bevorzugt 0,01-0,05 Mol Katalysator, bezogen auf 1 Mol Milchsäure.

Nach Neutralisation und Wäsche des Reaktionsgemisches schließt sich normalerweise eine Reinigung mittels Destillation an, die den MME in einer für die meisten Anwendungen geeigneten Reinheit (97 bis 98 %) liefert. Neben MME enthält das Destillat D-Milchsäure-L-menthylester (1-2 %) und L-Menthol (etwa 1 %). Beim Lagern über mehrere Wochen entwickelt das Produkt einen stechenden Geruch, der es für die meisten Verwendungszwecke unbrauchbar macht. Die Gründe für diese Produktveränderung sind nicht bekannt.

Überraschenderweise wurde nun gefunden, daß diese unerwünschte Produktveränderung in Gegenwart von Alkali- und/oder Erdalkalicarbonat und/oder -hydrogencarbonat nicht eintritt.

Gegenstand der Erfindung ist also eine Mischung aus
A. L-Milchsäure-L-menthylester und, pro Gewichtsteil A,
B. 0,0001 bis 0,05 Gewichtsteilen Alkali- und/oder Erdalkalicarbonat und/oder - hydrogencarbonat.

Die erfindungsgemäßen Mischungen sind über mehrere Monate lagerstabil; geruchliche Veränderungen beim Lagern sind nicht festzustellen.

Die erfindungsgemäßen Mischungen lassen sich auf jede erdenkliche Art herstellen, wie z.B. durch trockenes Mischen beider Komponenten (vorzugsweise in Pulverform) oder durch Lösen der Komponenten in einem für beide Bestandteile geeigneten Lösungsmittel wie wäßriges Methanol oder wäßriges Aceton und Abziehen des Lösungsmittels.

Nach einer bevorzugten Ausführungsform wird der MME, vorzugsweise nach vorangegangener Destillation, in Gegenwart des Zusatzes B umkristallisiert, wobei das verwendete Lösungsmittel für MME nicht auch gleichzeitig ein Lösungsmittel für B sein braucht; mit anderen Worten: Es ist für die Zwecke der Erfindung nicht notwendig, daß sich der Zusatz B in gelöstem MME auflöst; man kann den Zusatz B im gelösten MME - natürlich vorzugsweise in fein verteilter Form - dispergieren und dann zusammen mit den MME-Kristallen abtrennen.

Ein bevorzugtes Lösungsmittel für MME ist Aceton, wobei das Gewichtsverhältnis Lösungsmitte/MME für die Kristallisation vorzugsweise 0.5 bis 10, vorzugsweise 1 bis 3, beträgt.

Als Zusatz B kommen vorzugsweise Natriumcarbonat und -hydrogencarbonat sowie Kaliumcarbonat und -hydrogencarbonat oder Calciumcarbonat in Frage.

Die erfindungsgemäßen Mischungen können für die meisten Zwecke eingesetzt werden, ohne daß der Zusatz B vorher abgetrennt wird.

Die Prozentangaben des nachfolgenden Beispiels beziehen sich jeweils auf das Gewicht; Teile sind Gewichtsteile.

### Beispiel

1000 Teile destillierter MME, laut Gashromatogramm enthaltend
- 97,3 %: L-Milchsäure-L-menthylester,
- 1,4 %: D-Milchsäure-L-menthylester und
- 0,8 %: L-Menthol,
wurden in 500 Teilen Aceton, dem 1 Teil Natriumhydrogencarbonat zugesetzt war, bei 40°C gelöst. Es wird im Eisbad gekühlt; bei 12°C werden Impfkristalle zugegeben. Danach wird innerhalb von 2 Stunden auf 5°C abgekühlt (unter Rühren). Der Kristallbrei wird abfiltriert und an der Luft getrocknet.

Das erhaltene Produkt enthält laut Gaschromatogramm 99,7 % L-Milchsäure-L-menthylester. Es kann über Monate gelagert werden, ohne daß sich seine sensorischen Eigenschaften ändern.

## Patentansprüche

1. Mischung aus
A. L-Milchsäure-L-menthylester und, pro Gewichtsteil A,
B. 0,0001 bis 0,05 Gewichtsteilen Alkali- und/oder Erdalkalicarbonat und/oder - hydrogencarbonat.

## Claims

1. Mixture of
A. L-Lactic acid L-menthyl ester and, per part by weight of A,
B. 0.0001 to 0.05 part by weight of alkali metal carbonate and/or bicarbonate and/or alkaline earth metal carbonate and/or bicarbonate.

## Revendications

1. Mélange constitué
A. de L-lactate de L-menthyle et par partie en poids de A,
B. de 0,0001 à 0,-05 partie en poids de carbonate et/ou d'hydrogénocarbonate d'alcali et/ou de base alcalino-terreuse.
